# EUROPÄISCHE PATENTSCHRIFT

(11) **EP 1 055 725 B1**
(45) Veröffentlichungstag und Bekanntmachung des Hinweises auf die Patenterteilung: **22.10.2003**
(21) Anmeldenummer: 00109842.5
(22) Anmeldetag: 10.05.2000
(51) Int. Cl.: C12N 9/10, C12N 15/54, C12N 1/21, C12N 15/77, C12P 13/08

(54) **Verfahren zur fermentativen Herstellung von L-Aminosäuren und für das accDA Gen codierende Nukleotidsequenzen**
Method for the fermentative preparation of L-amino acids and nucleotide sequences coding for the accDA gene
Procédé de production de L-aminoacides par fermentation de bactéries et séquences nucléotides codantes pour le gene accDA

(30) Priorität: 27.05.1999 DE 19924365
(43) Veröffentlichungstag der Anmeldung: 29.11.2000
(73) Patentinhaber: Degussa AG, 40474 Düsseldorf (DE); Forschungszentrum Jülich GmbH, 52425 Jülich (DE)
(72) Erfinder: Tilg, Yvonne, 40822 Mettmann (DE); Eggeling, Lothar, Dr., 52428 Jülich (DE); Eikmanns, Bernhard, Prof. Dr., 89081 Ulm (DE); Sahm, Hermann, Prof. Dr., 52428 Jülich (DE); Möckel, Bettina, Dr., 40597 Düsseldorf (DE)

(56) Entgegenhaltungen:
- EP-A- 0 197 335
- EP-A- 0 358 940
- EP-A- 0 752 472
- EIKMANNS BERNHARD J ET AL: "Nucleotide sequence, expression and transcriptional analysis of the Corynebacterium glutamicum gltA gene encoding citrate synthase." MICROBIOLOGY (READING), Bd. 140, Nr. 8, 1994, Seiten 1817-1828, XP000957470
- JAGER ET AL., : "A Corynebacterium glutamicum gene encoding a two-domain protein similar to biotin carboxylases and biotin-carboxyl-carrier proteins" ARCH. MICROBIOL., Bd. 166, Nr. 2, 1996, Seiten 76-82, XP000957474
- CREMER J ET AL: "CONTROL OF THE LYSINE BIOSYNTHESIS SEQUENCE IN CORYNEBACTERIUM GLUTAMICUM AS ANALYZED BY OVEREXPRESSION OF THE INDIVIDUAL CORRESPONDING GENES" APPLIED AND ENVIRONMENTAL MICROBIOLOGY,US,WASHINGTON,DC, Bd. 57, Nr. 6, 1. Juni 1991 (1991-06-01), Seiten 1746-1752, XP000616281 ISSN: 0099-2240
- EGGELING L ET AL: "IMPROVED L-LYSINE YIELD WITH CORYNEBACTERIUM GLUTAMICUM: USE OF DAPA RESULTING IN INCREASED FLUX COMBINED WITH GROWTH LIMITATION" APPLIED MICROBIOLOGY AND BIOTECHNOLOGY,SPRINGER VERLAG, BERLIN,DE, Bd. 49, Nr. 1, 1998, Seiten 24-30, XP000918549 ISSN: 0175-7598
- EIKMANNS B J ET AL: "MOLECULAR ASPECTS OF LYSINE, THREONINE, AND ISOLEUCINE BIOSYNTHESIS IN CORYNEBACTERIUM GLUTAMICUM" ANTONIE VAN LEEUWENHOEK,DORDRECHT,NL, Bd. 64, Nr. 2, 1993, Seiten 145-163, XP000918559
- PATEK M ET AL: "Identification and transcriptional analysis of the dapB-ORF2-dapA-ORF4 operon of Corynebacterium glutamicum, encoding two enzymes involved in L-lysine synthesis." BIOTECHNOLOGY LETTERS, Bd. 19, Nr. 11, November 1997 (1997-11), Seiten 1113-1117, XP000956453 ISSN: 0141-5492

## Beschreibung

Gegenstand der Erfindung sind Verfahren zur fermentativen Herstellung von L-Aminosäuren, insbesondere L-Lysin unter Verwendung von coryneformen Bakterien, in denen das Gen accDA oder dafür codierende Nukleotidsequenzen mit der Herkunft Corynebacterium überexprimiert werden.

### Stand der Technik

L-Aminosäuren, insbesondere L-Lysin finden in der Tierernährung, in der Humanmedizin und in der pharmazeutischen Industrie Anwendung.

Es ist bekannt, daß diese Aminosäuren durch Fermentation von Stämmen coryneformer Bakterien insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Aminosäuren sind und L-Aminosäuren produzieren.

Seit einigen Jahren werden ebenfalls Methoden der rekombinanten DNA-Technik zur Stammverbesserung L-Aminosäure produzierender Stämme von Corynebacterium eingesetzt, indem man einzelne Aminosäure-Biosynthesegene amplifiziert und die Auswirkung auf die L-Lysin-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)) und Sahm et al. (Annuals of the New York Academy of Science 782, 25-39 (1996))

Das Enzym Acetyl-CoA Carboxylase katalysiert die Carboxylierung von Acetyl-CoA zu Malonyl-CoA. Das Enzym von Escherichia coli besteht aus vier Untereinheiten. Das accB-Gen kodiert für das Biotin-Carboxyl-Trägerprotein, das accC-Gen für die Biotin-Carboxylase und die Gene accA und accD für die Transcarboxylase (Cronan and Rock, Biosynthesis of Membrane lipids. In: Escherichia coli and Salmonella typhimurium (ed F.C. Neidhardt), 1996, pp 612-636, American Society for Microbiology). Wegen der Eigenschaft des Enzyms Acyl-Gruppen in Form des Acyl-CoA zu carboxylieren wird es auch als Acyl-CoA-Carboxylase bezeichnet.

Die Nukleotidsequenz des accBC-Gens von Corynebacterium glutamicum wurde von Jäger et al. (Archives of Microbiology 166,76-82 (1996)) bestimmt und ist bei der Datenbank der European Molecular Biologies Laboratories (EMBL, Heidelberg, Deutschland)unter der Accession Number U35023 allgemein verfügbar. Das accBC-Gen kodiert für eine Untereinheit der Acetyl-CoA Carboxylase, die eine Biotin-Carboxyl-Trägerprotein-Domäne und eine Biotin-Carboxylase-Domäne trägt.

### Aufgabe der Erfindung

Die Erfinder haben sich zur Aufgabe gestellt, neue Maßnahmen zur verbesserten fermentativen Herstellung von L-Aminosäuren, insbesondere L-Lysin bereitzustellen.

### Beschreibung der Erfindung

L-Aminosäuren finden in der Tierernährung, in der Humanmedizin und in der pharmazeutischen Industrie Anwendung. Es besteht daher ein allgemeines Interesse daran neue verbesserte Verfahren zur Herstellung von L-Aminosäuren bereitzustellen.

Wenn im folgenden L-Lysin oder Lysin erwähnt wird, sind damit nicht nur die Base sondern auch die Salze wie z. B. Lysin-Monohydrochlorid oder Lysin-Sulfat gemeint.

Beschrieben wird eine in coryneformen Mikroorganismen replizierbare, bevorzugt rekombinante DNA mit der Herkunft Corynebacterium, die zumindest die Nukleotidsequenz enthält, die für das accDA-Gen, dargestellt in der SEQ-ID-No. 1, codiert.

Die Erfindung betrifft ein Verfahren zur fermentativen Herstellung von L-Aminosäuren unter Verwendung von coryneformen Bakterien, die insbesondere bereits die L-Aminosäuren produzieren und in denen die für das accDA-Gen codierenden Nukleotidsequenzen überexprimiert werden.

Gegenstand der Erfindung ist schließlich auch ein Verfahren zur überexpression der Acyl-CoA-Carboxylase in coryneformen Bakterien durch gemeinsame Überexpression des erfindungsgemäß eingesetzten accDA-Gens und des bekannten accBC-Gens.

Die Mikroorganismen, die in dem erfindungsgemäßen Verfahren verwendet werden, können L-Aminosäuren aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463 und
Corynebacterium glutamicum FERM-P 6464

Die Erfinder isolierten das accDA-Gen von C. glutamicum Zur Isolierung des accDA-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 Stamm NM554 (Raleigh et al., 1988, Nucleic Acids Research 16:1563-1575) angelegt wurde. Börmann et al. (Molecular Microbiology 6(3), 317-326)) wiederum beschreiben eine Genbank von C. glutamicum ATCC13032 unter Verwendung des Cosmids pHC79 (Hohn und Collins, Gene 11, 291-298 (1980)).Zur Herstellung einer Genbank von C. glutamicum in E. coli können auch Plasmide wie pBR322 (Bolivar, Life Sciences, 25, 807-818 (1979)) oder pUC9 (Viera et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige für die Sequenzierung geeignete Vektoren subkloniert und anschließend sequenziert werden, so wie es z.B. bei Sanger et al. (Proceedings of the National of Sciences of the United States of America USA, 74:5463-5467, 1977) beschrieben ist.

Auf diese Weise wurde die für das Gen accDA kodierende DNA-Sequenz von C. glutamicum erhalten, die als SEQ ID NO 1 Bestandteil der vorliegenden Beschreibung ist. In SEQ ID NO 2 ist die Kodierregion (cds) des accDA-Gens wiedergegeben. Weiterhin wurde aus der vorliegenden DNA-Sequenz mit den oben beschriebenen Methoden die Aminosäuresequenz des entsprechenden Proteins abgeleitet. In SEQ ID NO 3 ist die sich ergebende Aminosäuresequenz des accDA-Genproduktes dargestellt.

Die Erfinder fanden heraus daß coryneforme Bakterien nach Überexpression der accDA Gene mit der Herkunft Corynebacterium in verbesserter Weise L-Lysin produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen L-Lysin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

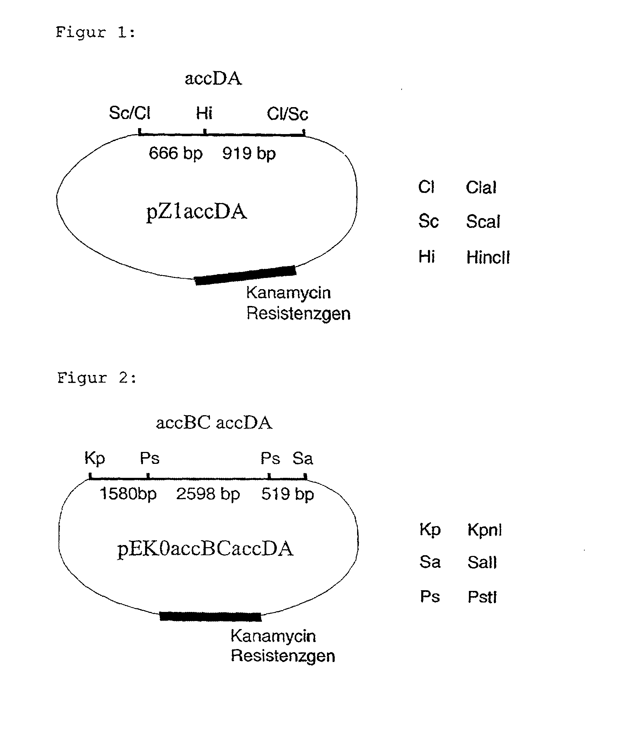

Ein Beispiel für ein Plasmid mit Hilfe dessen das accDA-Gen überexprimiert werden kann ist pZlaccDA (Figur 1), das in dem Stamm MH20-22B/pZ1accDA enthalten ist. Plasmid pZlaccDA ist ein auf Plasmid pZ1 (Menkel et al., Applied and Environmental Microbiology 55(3), 684-688 (1989)) basierender E. coli - C. glutamicum Pendelvektor, der das accDA-Gen trägt. Andere in C. glutamicum replizierbare Plasmidvektoren wie z.B. pEKEx1 (Eikmanns et al., Gene 102:93-98 (1991)) oder pZ8-1 (Europäische Patentschrift 0 375 889) können in gleicher Weise verwendet werden.

Die Erfinder fanden weiterhin heraus, daß durch zusätzliche Überexpression des bekannten accBC-Gens zusätzlich zum accDA-Gen coryneforme Bakterien in verbesserter Weise Acyl-CoA-Carboxylase produzieren. Ein Beispiel für ein Plasmid mit Hilfe dessen das accDA-Gen und das accBC-Gen gemeinsam überexprimiert werden können ist pEK0accBCaccDA (Figur 2). Plasmid pEK0accBCaccDA ist ein auf Plasmid pEK0 (Eikmanns et al., Gene 102, 93-98, (1991))basierender E. coli - C. glutamicum Pendelvektor, der das accBC- und das accDA-Gen trägt. Andere in C. glutamicum replizierbare Plasmidvektoren wie z.B. pEKEx1 (Eikmanns et al., Gene 102:93-98 (1991)) oder pZ8-1 (Europäische Patentschrift 0 375 889) können in gleicher Weise verwendet werden.

Zusätzlich kann es für die Produktion von L-Aminosäuren vorteilhaft sein neben dem accDA-Gen ein oder mehrere Enzyme des Biosyntheseweges zu überexprimieren. So kann beispielsweise für die Herstellung von L-Lysin
- gleichzeitig das für die Dihydrodipicolinat-Synthase kodierende dapA-Gen überexprimiert werden (EP-B 0 197 335), oder
- gleichzeitig ein S-(2-Aminoethyl)-Cystein-Resistenz vermittelndes DNA-Fragment amplifiziert werden (EP-A 0 088 166).

Weiterhin kann es für die Produktion von L-Aminosäuren, insbesondere L-Lysin vorteilhaft sein neben der Überexpression des accDA-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die in dem erfindungsgemäßen Verfahren verwendeten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Lysin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum der gewünschten L-Aminosäure gebildet hat. Dieses Ziel wird normalerweise innerhalb von 10 Stunden bis 160 Stunden erreicht.

Die Analyse von L-Lysin erfolgt kann durch Anionenaustauschchromatographie mit anschließender Ninhydrin Derivatisierung erfolgen so wie bei Spackman et al. beschrieben (Analytical Chemistry, 30, (1958), 1190).

Folgende Mikroorganismen wurden bei der Deutschen Sammlung für Mikrorganismen und Zellkulturen (DSMZ, Braunschweig, Deutschland) gemäß Budapester Vertrag hinterlegt:
- Corynebacterium glutamicum Stamm DSM5715/pZ1accDA als DSM 12785
- Corynebacterium glutamicum Stamm DSM5715/pEKOaccBCaccDA als DSM 12787

Das erfindungsgemäße Verfahren dient zur fermentativen Herstellung von L-Aminosäuren, insbesondere L-Asparaginsäure, L-Asparagin, L-Homoserin, L-Threonin, L-Isoleucin und L-Methionin mit coryneformen Bakterien, insbesondere der Herstellung von L-Lysin.

### Beispiele

Die vorliegende Erfindung wird im folgenden anhand von Ausführungsbeispielen näher erläutert.

### Beispiel 1

### Klonierung und Sequenzierung des accDA-Gens

Eine Genbank von C. glutamicum ATCC13032 wurde unter Verwendung des Cosmids pHC79 (Hohn und Collins, Gene 11, 291-298 (1980)), so wie bei Börmann et al. (Molecular Microbiology 6(3), 317-326)) beschrieben, hergestellt.

Ein ausgewähltes Cosmid wurde mit den Restriktionsenzymen EcoRI und XhoI nach Angaben des Herstellers dieser Restriktionsenzyme (Boehringer Mannheim) verdaut. Die entstandenen DNA-Fragmente wurden mit dem Vektor pUC18 (Norrander et al. (1983) Gene 26: 101-106), der ebenfalls mit den Restriktionsenzymen EcoRI und XhoI behandelt worden war, gemischt und nach Behandlung mit T4-DNA-Ligase in den E. coli Stamm DH5αmcr (Grant et al. (1990) Proceedings of the National Academy of Sciences, USA, 87: 4645-4645) kloniert, so wie bei Sambrook et al. (Molecular Cloning a Laboratory Manual (1989) Cold Spring Harbour Laboratories) beschrieben. Die Selektion der Transformanten erfolgte durch Selektion auf 50 µg/ml Ampicillin enthaltendem LB-Agar wie bei Sambrook et al. (Molecular Cloning a Laboratory Manual (1989) Cold Spring Harbour Laboratories) beschrieben. Aus einer Transformante wurde Plasmid-DNA isoliert und als pUCaccDA bezeichnet. Anschließend wurden mit dem von der Firma Promega (Heidelberg, Deutschland) für diesen Zweck vorgesehenen Kit (Erase-a-Base) über ExonukleaseIII-Verdau Subklone hergestellt. Diese wurden nach der Dideoxy-Kettenabbruchmethode von Sanger et al. sequenziert (Proceedings of the National Academy of Sciences USA (1977) 74: 5463-5467). Dabei wurde der Auto-Read Sequencing kit verwendet (Amersham Pharmacia Biotech, Uppsala, Schweden). Die gelelektrophoretische Analyse erfolgte mit dem automatischem Laser-Fluoreszenz Sequenziergerät (A.L.F.) von Amersham Pharmacia Biotech (Uppsala, Schweden). Die erhaltene Nukleotidsequenz wurde mit dem Programmpaket HUSAR (Release 4.0, EMBL, Heidelberg, Deutschland) analysiert. Die Nukleotidsequenz ist in SEQ ID NO 1 wiedergegeben. Die Analyse der Nukleotidsequenz ergab ein offenes Leseraster von 1473 Basenpaaren, welches als accDA-Gen bezeichnet wurde. Das accDA Gen aus C. glutamicum kodiert für ein Polypeptid von 484 Aminosäuren.

### Beispiel 2

### Expression der accDA-Gens in Corynebacterium glutamicum

Das Gen accDA wurde zur Expression in C. glutamicum in den Vektor pZ1 (Menkel et al. (1989) Applied and Environmental Microbiology 55: 684-688) subkloniert. Dazu wurde das Plasmid pUCaccDA (Siehe Beispiel 1) mit dem Restriktionsenzym ClaI geschnitten. Das resultierende 1,6 kb große Fragment wurde wie in Beispiel 1 beschrieben isoliert, mit Klenow-Polymerase und alkalischer Phosphatase behandelt und zur Ligation mit pZ1 eingesetzt. Dieser Vektor war zuvor mit ScaI linearisiert worden. Mit dem Ligationsansatz wurde E. coli DH5αmcr (Grant et al. (1990) Proceedings of the National Academy of Sciences, USA, 87: 4645-4645) transformiert und Transformanten auf Kanamycin haltigem (50 µg/ml) LB-Agar selektioniert. Dadurch wurde der 7,7 kb große Pendelvektor pZlaccDA (Figur 1) erhalten. Dieser wurde mittels Elektroporation wie bei Haynes (FEMS Microbiol. Letters (1989) 61: 329-334) beschrieben in den Stamm DSM5715 eingebracht, wobei die Transformanten auf LBHIS-Agar selektioniert wurden (Liebl et al., FEMS Microbiology Letters 1989, 65: 299-304). Auf diese Weise wurde der C. glutamicum Stamm DSM5715/pZ1accDA erhalten.

### Beispiel 3

### Herstellung von L-Lysin mit dem Stamm DSM5715/pZ1accDA

Der Stamm DSM5715/pZ1accDA wurde nach Vorkultivierung in Medium CgIII (Keilhauer et al. 1993, Journal of Bacteriology 175:5595-5603), im Produktionsmedium CgXII (Keilhauer et al. 1993, Journal of Bacteriology 175:5595-5603) kultiviert. Es wurden 4% Glukose und 50 mg/l Kanamycinsulfat zugesetzt.

Nach 48 Stunden Inkubation wurde die optische Dichte bei 660nm und die Konzentration an gebildetem L-Lysin bestimmt In Tabelle 1 ist das Ergebnis des Versuches dargestellt.

**Tabelle 1**

| Stamm | OD | L-Lysin g/l |
|---|---|---|
| DSM5715 | 31,4 | 7,2 |
| DSM5715/pZ1accDA | 43,1 | 8,0 |

### Beispiel 4

### Gemeinsame Expression von accBC und accDA

(i) Konstruktion des Expressionsvektors pEK0accBCaccDA. Das das accBC enthaltende Plasmid pWJ71 (Jäger et al., Archives of Microbiology (1996) 166:76-82) wurde mit den Restriktionsenzymen AgeI und SmaI verdaut und anschließend mit Klenow-Polymerase und alkalischer Phosphatase behandelt. Parallel dazu, wurde das Plasmid pUCaccDA EcoRI/XhoI verdaut, und anschließend eine Behandlung mit Klenow-Polymerase und alkalischer Phosphatase durchgeführt. Durch präparative Isolation aus einem Agarosegel, die wie bei Sambrook et al. (Molecular Cloning a Laboratory Manual (1989) Cold Spring Harbour Laboratories) beschrieben durchgeführt wurde, wurde das 2,1 kb große accDA tragende Fragment isoliert. Dieses wurde mit dem Vektor pWJ71, der wie zuvor beschrieben vorbereitet war, ligiert. Aus dem resultierenden Plasmid wurde das 4,6 kb große accBCaccDA-tragenden Fragment durch KpnI/SalI Verdau ausgeschnitten, und wieder präparativ durch Agarosegelelektrophorese isoliert. Zur Ligation dieses Fragments mit dem C. glutamicum/E. coli shuttle Vektor pEK0 (Eikmanns et al. (1991) Gene 102: 93-98) wurde pEK0 mit den Restriktionsenzymen KpnI und SalI verdaut, und anschließend eine Behandlung mit Klenow-Polymerase und alkalischer Phosphatase durchgeführt. Der so vorbereitete Vektor wurde mit dem 4,6 kb großen accBCaccDA-tragenden Fragment ligiert. Der resultierende Vektor pEK0accBCaccDA ist in Figur 2 dargestellt. Dieser Vektor wurde mittels Elektroporation (Haynes 1989, FEMS Microbiol. Letters 61: 329-334) so wie in Beispiel 2 beschrieben in den Stamm ATCC13032 eingebracht. Auf diese Weise wurde der C. glutamicum Stamm ATCC13032/pEK0accBCaccDA erhalten.
(ii) Bestimmung der Acyl-CoA Carboxylase Aktivität.
Der Stamm C. glutamicum ATCC13032/pEK0accBCaccDA wurde nach Vorkultur in Medium CGIII (Keilhauer et al. 1993, Journal of Bacteriology 175:5595-5603) in dem Medium CGXII angezogen, das bei Keilhauer et al. (Journal of Bacteriology (1993) 175: 5595-5603) beschrieben ist. Die Zellen wurden durch Zentrifugation geerntet, und das Zellpellet wurde einmal mit 60 mM Tris-HCl (pH 7,2) gewaschen und im selben Puffer resuspensiert. Der Zellaufschluß erfolgte mittels 10 minütiger Ultraschallbehandlung (Branson-Sonifier W-250, Branson Sonic Power Co, Danbury, USA). Anschließend wurden die Zelltrümmer durch eine 30 minütige Zentrifugation bei 4 °C abgetrennt und der Überstand als Rohextrakt in den Enzymtest eingesetzt. Der Reaktionsansatz des Enzymtests enthielt in 1 ml Reaktionsvolumen 60 mM Tris-HCl (pH 7,2), 65 mM KHCO₃, 1 mM ATP, 1,5 mM MgCl₂, 4 mM AcylCoA (wahlweise Acetyl-CoA oder Propionyl-CoA) und 4 mg Rohextrakt. Die Testansätze wurden bei 30°C inkubiert und nach 15, 30, 45 und 60 Sekunden 100µl Proben genommen und deren Malonyl- bezw. Methylmalonyl-CoA Konzentration mittels HPLC-Analytik bestimmt (Kimura et al. (1997) Journal of Bacteriology 179: 7098-7102). Wie Tabelle 2 zeigt, weist der Stamm C. glutamicum ATCC13032/pEKOaccBCaccDA eine hohe Acyl-CoA Carboxylase Aktivität mit Acetyl-CoA und auch mit Propionyl-CoA auf, wogegen der Kontrollstamm nur eine geringe Acyl-CoA Carboxylase Aktivität mit Acetyl-CoA und auch mit Propionyl-CoA hat.

**Tabelle 2**

| Spezifische Acyl-CoA Carboxylase Aktivität (µmol/min und mg Protein) in C. glutamicum | | |
|---|---|---|
| | Acyl-CoA Carboxylase-Aktivität mit dem Substrat | |
| Stamm | Acetyl-CoA | Propionyl-CoA |
| ATCC13032/pEK0accBCaccDA | 0,048 | 0,124 |
| ATCC13032/pEK0 | 0,011 | 0,018 |

Folgende Figuren sind beigefügt:
- Figur 1: Karte des Plasmids pZ1accDA.
- Figur 2: Karte des Plasmids pEK0accBCaccDA

## Patentansprüche

1. Verfahren zur Herstellung von L-Aminosäuren durch Fermentation coryneformer Bakterien,
**dadurch gekennzeichnet,**
**daß** man Bakterien einsetzt, in denen man das accDA-Gen oder dafür codierende Nukleotidsequenzen mit der Herkunft Corynebacterium überexprimiert.

2. Verfahren gemäß Anspruch 1,
**dadurch gekennzeichnet,**
**daß** man einen mit einem Plasmidvektor transformierten Stamm einsetzt und der Plasmidvektor die für das accDA-Gen mit der Herkunft Corynebacterium codierende Nukleotidsequenz trägt.

3. Verfahren gemäß Anspruch 2,
**dadurch gekennzeichnet,**
**daß** man mit dem Plasmidvektor pZ1accDA, hinterlegt in Corynebacterium glutamicum unter der Nummer DSM 12785, transformierte Bakterien einsetzt.

4. Verfahren gemäß einem oder mehreren der Ansprüche 1 bis 3,
**dadurch gekennzeichnet,**
**daß** man coryneforme Bakterien verwendet, die L-Asparaginsäure, L-Asparagin, L-Homoserin, L-Threonin, L-Isoleucin und L-Methionin herstellen.

5. Verfahren gemäß einem oder mehreren der Ansprüche 1 bis 3,
**dadurch gekennzeichnet,**
**daß** man coryneforme Bakterien verwendet, die L-Lysin herstellen.

6. Verfahren gemäß einem oder mehreren der Ansprüche 1 bis 5,
**dadurch gekennzeichnet,**
**daß** man das accBC-Gen mit der Herkunft Corynebacterium glutamicum zusätzlich zum accDA-Gen mit der Herkunft Corynebacterium überexprimiert.

7. Verfahren gemäß Anspruch 1,
**dadurch gekennzeichnet,**
**daß** gleichzeitig das für die Dihydrodipicolinat-Synthase kodierende dapA-Gen mit der Herkunft Corynebacterium glutamicum überexprimiert wird.

8. Verfahren gemäß Anspruch 1,
**dadurch gekennzeichnet,**
**daß** gleichzeitig ein S-(2-Aminoethyl)-Cystein-Resistenz vermittelndes DNA-Fragment mit der Herkunft Corynebacterium glutamicum amplifiziert wird.

9. Verfahren zur fermentativen Herstellung von L-Aminosäuren gemäß einem oder mehreren der Ansprüche 1 bis 8,
**dadurch gekennzeichnet,**
**daß** man folgende Schritte durchführt:
a) Fermentation der die gewünschten L-Aminosäure produzierenden coryneformen Bakterien, in denen zumindest das accDA-Gen mit der Herkunft Corynebacterium glutamicum überexprimiert wird.
b) Anreicherung der gewünschten L-Aminosäure im Medium oder in den Zellen der Bakterien und
c) Isolieren der gewünschten L-Aminosäure.

## Claims

1. Process for the preparation of L-amino acids by the fermentation of corynebacteria, **characterized in that** bacteria are used in which the accDA gene or nucleotide sequences coding therefor, originating from Corynebacterium, are overexpressed.

2. Process according to Claim 1, **characterized in that** a strain transformed with a plasmid vector is used and the plasmid vector carries the nucleotide sequence coding for the accDA gene originating from Corynebacterium.

3. Process according to Claim 2, **characterized in that** bacteria transformed with plasmid vector pZ1accDA, deposited in Corynebacterium glutamicum under the number DSM 12785, are used.

4. Process according to one or more of Claims 1 to 3, **characterized in that** corynebacteria are used which produce L-aspartic acid, L-asparagine, L-homoserine, L-threonine, L-isoleucine and L-methionine.

5. Process according to one or more of Claims 1 to 3, **characterized in that** corynebacteria are used which produce L-lysine.

6. Process according to one or more of Claims 1 to 5, **characterized in that** the accBC gene originating from Corynebacterium glutamicum is overexpressed in addition to the accDA gene originating from Corynebacterium.

7. Process according to Claim 1, **characterized in that** the dapA gene originating from Corynebacterium glutamicum and coding for dihydrodipicolinate synthase is simultaneously overexpressed.

8. Process according to Claim 1, **characterized in that** a DNA fragment originating from Corynebacterium glutamicum and conferring S-(2-aminoethyl)cysteine resistance is simultaneously amplified.

9. Process for the preparation of L-amino acids by fermentation according to one or more of Claims 1 to 8, **characterized in that** the following steps are carried out:
a) fermentation of the corynebacteria producing the desired L-amino acid, in which at least the accDA gene originating from Corynebacterium glutamicum is overexpressed,
b) enrichment of the desired L-amino acid in the medium or in the cells of the bacteria, and
c) isolation of the desired L-amino acid.

## Revendications

1. Procédé de production de L-aminoacides par fermentation de bactéries coryneformes,
**caractérisé en ce qu'**
on met en oeuvre des bactéries dans lesquelles on surexprime le gène accDA ou des séquences de nucléotides qui codent pour celui-ci, ayant la provenance corynebactérium.

2. Procédé conformément à la revendication 1,
**caractérisé en ce qu'**
on met en oeuvre une souche transformée par un vecteur plasmidique et le vecteur plasmidique porte la séquence de nucléotide codant pour le gêne accDA ayant la provenance corynebactérium.

3. Procédé conformément à la revendication 2,
**caractérisé en ce qu'**
on met en oeuvre des bactéries transformées avec le vecteur plasmidique pZlaccDA déposé dans corynebacterium glutamicum, sous le numéro DSM 12785.

4. Procédé conformément à l'une ou plusieurs des revendications 1 à 3,
**caractérisé en ce qu'**
on utilise des bactéries coryneformes qui produisent de l'acide L-aspartique, de la L-asparagine, de la L-homosérine, de la L-thréonine, de la L-isoleucine et de la L-méthionine.

5. Procédé conformément à l'une ou plusieurs des revendications 1 à 3,
**caractérisé en ce qu'**
on utilise des bactéries coryneformes qui produisent de la L-lysine.

6. Procédé conformément à l'une ou plusieurs des revendications 1 à 5,
**caractérisé en ce qu'**
on surexprime le gêne accBC ayant la provenance corynebacterium glutamicum en supplément au gêne accDA ayant la provenance corynebacterium.

7. Procédé conformément à la revendication 1,
**caractérisé en ce qu'**
on surexprime en même temps le gêne dapA qui code pour la dihydrodipicolinate -synthase et ayant la provenance corynebacterium glutamicum.

8. Procédé conformément à la revendication 1,
**caractérisé en ce qu'**
on amplifie en même temps un fragment d'ADN ayant la provenance corynebacterium glutamicum et qui procure la résistance à la S-(2-aminoéthyl)cystéine.

9. Procédé de production par fermentation de L-aminoacides conformément à l'une ou plusieurs des revendications 1 à 8,
**caractérisé en ce qu'**
on effectue les étapes suivantes :
a) fermentation des bactéries coryneformes qui produisent le L-aminoacide désiré dans lesquelles au moins le gêne accDA ayant la provenance corynebacterium glutamicum est surexprimé,
b) enrichissement du L-aminoacide désiré dans le milieu ou dans les cellules des bactéries et
c) isolement du L-aminoacide désiré.
